# EUROPEAN PATENT APPLICATION

(11) **EP 4 159 737 A1**
(43) Date of publication of application: **05.04.2023**
(21) Application number: 21813921.0
(22) Date of filing: 26.05.2021
(51) Int. Cl.: C07D 491/06

(54) **METHOD FOR PREPARING GLP-1 RECEPTOR AGONIST FREE BASE**

(30) Priority: 28.05.2020 CN 202010465802
(71) Applicant: Hangzhou Zhongmeihuadong Pharmaceutical Co., Ltd., GongShu District HangZhou, Zhejiang 310011 (CN)
(72) Inventor: HU, Haiwen, HangZhou, Zhejiang 310011 (CN); FU, Hongliang, HangZhou, Zhejiang 310011 (CN); CHEN, Fenfen, HangZhou, Zhejiang 310011 (CN); ZHOU, Xinjie, HangZhou, Zhejiang 310011 (CN)
(74) Representative: Mewburn Ellis LLP
(86) International application number: PCT/CN2021/095969
(87) International publication number: WO 2021/238964

(57) **Abstract**

The invention relates the preparation method of a free base GLP-1 receptor agonist, specifically relates to preparation of (S)-2-(3S,8S)-3-(4-(3,4-dichlorobenzyloxy)phenyl-7-((S)-1- phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base. First, compound III is reacted with a condensation agent to give an active ester, and the amino organic acid salt is dissociated in situ and then directly involved in the amide condensation. Subsequently, in the hydrolysis, by means of the workup mode of acid-base neutralization, (S)-2-(3S,8S)-3-(4-(3,4-dichlorobenzyloxy)phenyl-7-((S)-1- phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base is precipitated from suitable solvent in high purity. The method comprising two steps achieves a yield above 80%, and the purity of (S)-2-(3S,8S)-3-(4-(3,4-dichlorobenzyloxy)phenyl-7-((S)-1- phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base is above 98%. Moreover, the method can achieve large-scale preparation and is suitable for industrial production.

## Description

### Technical Field

The invention belongs to the technical field of medicine, and particularly relates to a method for preparing (S)-2-(3S,8S)-3-(4-(3,4-dichlorobenzyloxy)phenyl-7-((S)-1- phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid.

### Background of the Invention

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride is an oral, non-peptide glucagon-like peptide 1 (GLP-1) receptor agonist with high selectivity, and now is conducting clinical research.

TTP273 has a molecular formula of C₅₀H₄₉Cl₄N₃O₆, a molecular weight of 929.76, and the following chemical structure:

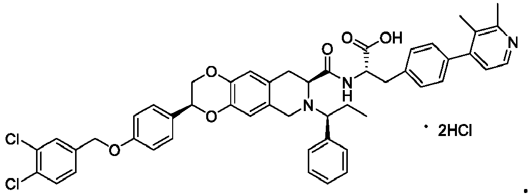

A patent for invention CN102378574B discloses a method for preparing (S)-2-(3S,8S)-3-(4-(3,4-dichlorobenzyloxy)phenyl-7-((S)-1-phenylpropyl)- 2,3,6,7,8,9-hexahydro-[1,4]-dioxino[2,3-g]isoquinolin-8-ylformylamino)-3-(4-(2,3-dimethylpyridin-4-yl)phenyl)propanoic acid, with the following specific scheme:

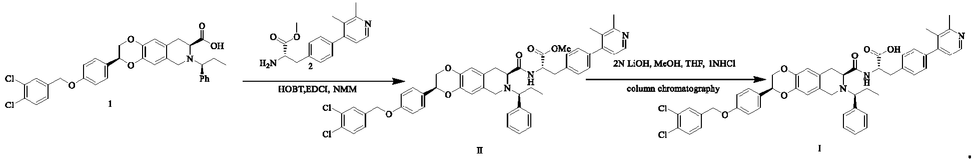

In the first step of amide condensation of the known scheme, as methyl (S)-2-amino-3-(4-(2,3-dimethylpyridin-4-yl)phenyl)propionate (compound 2) in the form of hydrochloride tends to absorb moisture and cannot be used directly, the actual form used in the reaction is the free base of compound 2. The free base of compound 2 is obtained as a viscous liquid through steps including the dissociation of the hydrochloride form of compound 2, multiple extraction, concentration and dryness. In practice, the multiple steps are tedious, and it is difficult to transfer the intermediates products and hard to quantify accurately. According to the available prior art inventions, the carboxylate form of compound 2 has increased stability, but is still difficult to directly use in amide condensation owning to the disturbance of the carboxylic acid. In addition, after the second step of hydrolysis, the scheme still requires column chromatographic purification to obtain (S)-2-(3S,8S)-3-(4-(3,4-dichlorobenzyloxy)phenyl-7-((S)-1-phenylpropyl)- 2,3,6,7,8,9-hexahydro-[1,4]-dioxino[2,3-g]isoquinolin-8-ylformylamino)-3-(4-(2,3-dimethylpyridin-4-yl)phenyl)propanoic acid free base.

In summary, the preparation method disclosed in the prior art has complex operations, requires column chromatographic purification and cannot achieve the industrial production of (S)-2-(3S,8S)-3-(4-(3,4-dichlorobenzyloxy)phenyl-7-((S)-1-phenylpropyl)- 2,3,6,7,8,9-hexahydro-[1,4]-dioxino[2,3-g]isoquinolin-8-ylformylamino)-3-(4-(2,3-dimethylpyridin-4-yl)phenyl)propanoic acid.

### Summary of the Invention

The invention provides a preparation method for preparing (S)-2-(3S,8S)-3-(4-(3,4-dichlorobenzyloxy)phenyl-7-((S)-1-phenylpropyl)- 2,3,6,7,8,9-hexahydro-[1,4]-dioxino[2,3-g]isoquinolin-8-ylformylamino)-3-(4-(2,3-dimethylpyridin-4-yl)phenyl)propanoic acid free base in large scale. The method utilizes an intermediate state of active ester, makes it possible for the organic acid salt of the amino acid derivative to directly participate in the amide condensation, and thus avoids tedious pretreatment operations. Furthermore, after the crystallization mode of acid-base neutralization, (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid free base is obtained in high purity, without the separation and purification by column chromatography.

The preparation method of the invention comprises the following steps:
a) reacting compound of formula III with a condensation agent to give an active ester X;
b) active ester X further reacting with organic acid salt IV (x is 0.5~3) to give compound of formula II: 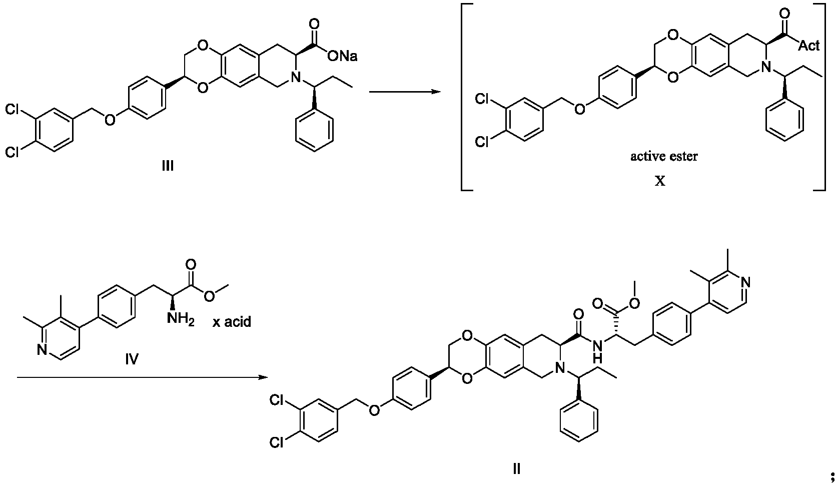
c) subjecting compound of formula II to hydrolysis and neutralization with an acid solution, to give compound of formula I:

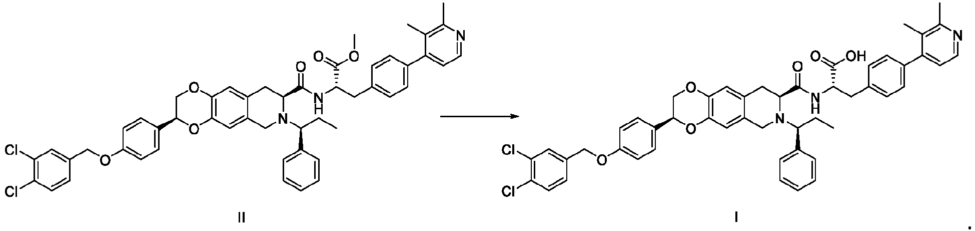

As a specific embodiment, in step a), compound III is first reacted with a condensation agent to give an active ester, wherein the condensation agent is one, two or more selected from the group consisting of HATU, HBTU, HCTU, PyBOP, and TBTU, and preferably PyBOP.

As a specific embodiment, in step a), the active ester X is selected from:

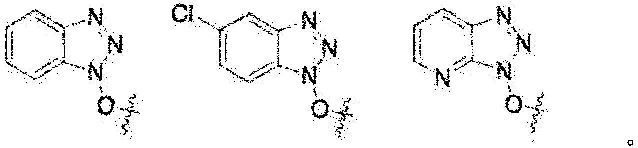

As a specific embodiment, in step b), the organic acid salt IV is the salt formed from methyl (S)-2-amino-3-(4-(2,3-dimethylpyridin-4-yl)phenyl)propionate (the above mentioned compound 2) with an organic acid, wherein the organic acid is one selected from the group consisting of oxalic acid, citric acid, L-tartaric acid, malic acid, succinic acid, maleic acid, fumaric acid, glycollic acid, and hippuric acid, and the molar ratio of compound 2 to the organic acid is 1:0.5^{~} 1:3.

As a specific embodiment, the reaction solvent in step a) and b) is one, two or more selected from the group consisting of N,N-dimethyl formamide, N,N-dimethyl acetamide, dimethyl sulfoxide, dichloromethane, trichloromethane, tetrahydrofuran, and 2-methyltetrahydrofuran, and preferably dimethyl sulfoxide.

As a specific embodiment, in step b), after the addition of organic acid salt IV, an alkaline reagent was addded, wherein the alkaline reagent is one, two or more organic bases selected from the group consisting of triethylamine, N-methylmorpholine, diisopropylethylamine, and 4-methylaminopyridine, and preferably triethylamine.

As a specific embodiment, in step a), compound III and the condensation agent are fed at a molar ratio in the range of 1:1.1^{~}1:2; and compound III, organic acid salt IV and the organic base are fed in a molar ratio in the range of 1:1.05:3^{~}1:1.1:5.

As a specific embodiment, in step a) and b), the reaction is carried out at a temperature in the range of 10^{~}50°C, and preferably 15^{~}35°C.

As a specific embodiment, in step b), the organic acid salt IV is selected from oxalate, citrate, L-tartrate, malate, succinate, maleate, fumarate, glycolate or hippurate.

As a specific embodiment, the workup mode of step b) is as follows: after the completion of reaction, ethyl acetate and water are added in a weight amount 8^{~}10 times to that of compound III, or water alone is added in a weight amount 8^{~}10 times to that of compound III, and the mixture is mixed homogeneously, extracted and washed; the aqueous layer is removed out, and the organic layer is concentrated to dryness and directly used in the next step, yield being recorded as 100%.

As a specific embodiment, in step c), the hydrolysis reaction is carried out by the addition of an aqueous solution of alkaline agent, wherein the alkaline reagent is one selected from lithium hydroxide, sodium hydroxide, and potassium hydroxide, and preferably potassium hydroxide, and the concentration of the aqueous solution is 1^{~}4 mol/L; and compound II and the alkaline reagent are fed at a molar ratio in the range of 1:2^{~}1:4.

As a specific embodiment, the reaction solvent in step c) is one, two or more selected from the group consisting of tetrahydrofuran, 2-methyltetrahydrofuran, acetonitrile, acetone, methanol, and ethanol, and preferably acetone or a mixed solvent of acetone and ethanol.

As a specific embodiment, the reaction solvent in step c) is mixed solvent of acetone and ethanol, the weight ratio of acetone to ethanol in the mixed solvent is in the range of 4:1^{~}1:1, and preferably 2:1.

As a specific embodiment, in step c), the weight amount of the solvent is 4^{~}8 times to that of compound II.

As a specific embodiment, in step c), the reaction mixture is neutralized with an acidic aqueous solution, wherein the acid is one selected from hydrochloric acid, hydrobromic acid, sulfuric acid, and phosphoric acid, and the concentration of the acidic aqueous solution is 1^{~}4 mol/L.

As a specific embodiment, the workup mode of step c) is as follows: after the neutralization with an acid, the mixture is stirred at 10^{~}30°C to crystalize for 16^{~}20 hours.

As a specific embodiment, the present invention provides the use of organic acid salts IV in step b) for the preparation of compounds of formula I.

As a specific embodiment, the present invention provides the use of compound I in step c) for the preparation of formula I compound dihydrochloride.

The steps of amide condensation and hydrolysis according to the method of the invention are key steps for the preparation of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride, and the quality of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base (compound of formula I) is crucial for the quality of the final product of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride. The method of the invention first reacts compound of formula III with a condensation agent to give an active ester, and then dissociates the amino organic acid salt in situ, which avoids the side reaction of the condensation of the free organic acid radical with the organic amine. The method of the invention makes it possible for the organic acid salt of the amino acid derivative to directly participate in the amide condensation, and thus avoids steps including dissociation, extraction and drying. Furthermore, by means of the workup mode of acid-base neutralization, (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base (compound of formula I) is precipitated from suitable solvent in high purity, and thus avoids the tedious steps such as extraction, concentration and column chromatography. The method of two steps achieves a yield above 80% and the purity of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base can be as high as above 98%.

### Detailed Description of the Invention

The invention will be further illustrated by combining the following examples. The following examples are used to explain the method of the invention and the core concept thereof, and for those skilled in the art, any possible change or substitution without departing from the inventive concept will fall within the protection scope of the invention. In the following examples, where the specific conditions of the experimental methods are not indicated, they are typically the conventional conditions, or are those recommended by the raw material or commodity manufactures; and the solvents without indicating the source are typically conventional solvents that are commercially available.

The detection instrument used in the present invention is:
1. NMR
   Instrument model: Bruker DMX-500 nuclear magnetic resonance instrument
2. Mass Spectrometer
   Instrument model: Agilent 6460, test conditions: ESI source

The term "yield is based on 100%" can be understood that there is no material loss during the preparation process, and the target product is completely converted and used for the next reaction.

Compound of formula III and compound 2 were prepared according to the methods described in CN102378574B, and the patent is hereby incorporated by reference in their entirety.

The preparation method of compound I disclosed in CN102378574B, the total yield of compound 1 to prepare the compound of formula I in two steps is about 15%, see paragraphs [1634] and [2081] of the description of CN102378574B.

The compound of formula III is first prepared by the method disclosed in patent CN102378574B to obtain carboxylic acid compound 1, compound 1 is redissolved in 10 times the mass of toluene, 10 times the mass of 4N NaOH is then added, stirred to form a salt, then the toluene layer is separated, and concentrated to obtain the compound III.

The preparation method of organic acid salt IV (taking oxalic acid salt as the example) was as follows: the free base of compound 2 was dissolved in acetone, wherein the weight of the acetone is 5 times to that of compound 2, and then a solution of oxalic acid in methanol was added; the mixture was stirred to crystalize, and the organic acid salt IV was obtained after filtration.

Salt IV (taking oxalic acid salt as the example), 1H NMR (500 MHz, d6-DMSO) δ 8.37 (d, J=5.0Hz, 1H), 7.38-7.16 (m, 4H), 7.15 (s, 1H), 4.35-4.32 (m, 1H), 3.26-3.15 (m, 2H), 3.70 (s, 3H), 2.56

(s, 3H), 2.53 (s, 3H); 13C NMR (100 MHz, d6-DMSO) δ D169.5,163.4(2C),158.5,157.8,148.9,145.9, 142.0, 141.8, 140.9, 138.7, 156.6, 149.7, 144.1, 137.6, 134.8, 129.5(2C), 129.4, 128.9(2C), 122.6, 53.2, 52.6, 35.7, 22.2, 15.8; HRMS (ESI) C17H20N2O2(as free base) calcd for, 285.1598 [M+H], found: 285.1604.

The preparation method of other organic acid salts is the same as the above-mentioned oxalate, that is, the free base of compound 2 is mixed in a solvent of 5 times the mass, and the solution of the corresponding organic acid is added, after adding, stirring and crystallization, and filtering to obtain.

### Example 1: Preparation of compound of formula II

Compound of formula III (62.6 g, 0.1 mol), DMSO (300 g) and PyBOP (57.2 g, 0.11 mol) were charged into a reaction flask, and stirred at 15^{~}25°C for 2 hours. TLC was used to monitor the time point when compound III disappeared. After the addition of organic acid salt IV (compound 2 dioxalate)(51.0 g, 0.11 mol), triethylamine (40.5 g, 0.4 mol) was added and stirred at 25°C for 2 hours. TLC was used to monitor the time point when the active ester intermediate state disappeared. Ethyl acetate (500.8 g) and purified water (626.0 g) were added and stirred for 30 minutes. The aqueous layer was removed out, and the organic layer was concentrated to dryness and directly used in the next step, yield being recorded as 100%. ¹H NMR (500 MHz, d6-DMSO) δ 8.27 (d, J=5.0Hz, 1H), 8.00 (d, J = 8.0Hz, 1H), 7.72 (d, J = 1.5Hz, 1H), 7.66 (d, J =8.0Hz, 1H), 7.45-7.43 (m, 1H), 7.33-7.30 (m, 2H), 7.27-7.19 (m, 7H), 7.98-6.91 (m, 5H), 6.68 (s, 1H), 6.53 (s, 1H), 5.12 (s, 1H), 5.10-5.08 (m, 1H), 4.73-4.69 (m, 1H), 4.30-4.27 (m, 1H), 3.84-3.80 (m, 1H), 3.67 (s, 3H), 3.57-3.55(m, 1H), 3.51-3.43 (m, 1H), 3.25-3.23 (m, 1H), 3.11-3.06 (m, 1H), 2.95-2.91 (m, 1H), 2.83-2.79 (m, 1H), 2.71-2.67 (m, 1H), 2.46 (s, 1H), 2.08 (s, 1H), 1.92-1.86 (m, 1H), 1.70-1.64 (m, 1H), 0.55 (t, J = 7.0 Hz, 3H); ¹³C NMR (100 MHz, d6-DMSO) δ 173.4, 171.9, 158.5, 157.8, 148.9, 145.9, 142.0, 141.8, 140.9, 138.7, 138.0, 136.6, 131.6, 131.2, 130.9, 129.9, 129.8, 129.6(2C), 129.5, 129.1(2C), 129.0(2C), 128.8, 128.7(2C), 128.5, 128.2(2C), 127.7, 122.6, 115.9, 115.2(2C), 114.9, 74.3, 68.7, 68.2, 68.1, 59.0, 52.7, 52.5, 46.6, 37.0, 29.3, 26.0, 23.6, 16.2, 11.2; MS (ESI) m/z (%): 870.2 (100) [M]+.

### Example 2: Preparation of compound of formula I ((S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base)

The freshly prepared compound of formula II (87.9 g, 0.1 mol) and a solution of acetone and ethanol (351.6 g and 175.8 g, respectively) were charged into a reaction flask, and then stirred homogeneously. A sodium hydroxide solution of 2 mol/L (15.8 g, 202 mL) was added, and the reaction was carried out at 15^{~}25°C. TLC was used to monitor the time point when compound II disappeared. Then, a hydrochloric acid solution of 1mol/L (39.6 g, 404 mL) was begun to be added, the mixture was stirred at 10^{~}30°C to crystalize for 16^{~}18 hours, and 68.4 g white solid compound was obtained after suction filtration, yield: 80%, purity: 98.4%. ¹H NMR (500 MHz, d5-Pyridine) δ 8.83 (d, J=8.0 Hz, 1H), 8.61 (dd, J=5.0, 1.5 Hz, 1H), 7.75 (s, 1H), 7.62 (s, 1H), 7.55-7.50 (m, 3H), 7.45 (d, J=8.0 Hz, 1H), 7.40-7.30 m, 7H), 7.30 (d, J = 8.0 Hz, 2H), 7.12 (dd, J=14.5, 6.5 Hz, 3H), 7.03 (s, 1H), 6.98 (s, 1H), 5.49-5.46 (m, 1H), 5.22 (d, J=9.0 Hz, 1H), 5.19 (s, 2H), 4.40 (d, J=11.0 Hz, 1H), 4.09 (d, J=5.0 Hz, 1H), 4.06-3.97 (m, 2H), 3.87 (dd, J = 10.0, 5.0 Hz, 1H), 3.72 (d, J =14.0 Hz, 1H), 3.54-3.50 (m, 1H), 3.49-3.42 (m, 2H), 3.12 (dd, J = 15.0, 7.5 Hz, 1H), 2.64 (s, 3H), 2.20-2.13 (m, 1H), 2.10 (s, 3H), 2.07-2.00 (m, 1H), 0.69 (t, J=7.5 Hz, 3H); HRMS (ESI) Calcd. For C₅₀H₄₇Cl₂N₃O₆ 856.2915 [M+H], found: 856.2905.

### Example 3^{~}7: Preparation of compound of formula I with various organic acid salts IV

Methods for preparing compound of formula I with various organic acid salts IV were provided, and the steps were substantially the same as those of Example 1 and Example 2, wherein the free base compound 2 and the organic acid in the organic acid salt IV were at the ratio of 1:1, and the fed amounts were the same as those in Example 1. The results were shown in Table 1.

**Table 1 Results of the preparation of compound of formula I with various organic acid salts**

| Example | Acid radical of IV | Fed ratio of III: PyBop: IV: triethylamine | Compound I Yield (%) | Compound I Purity (%) |
|---|---|---|---|---|
| 3 | citric acid | 1:1.1:1.1:4 | 78.9 | 98.1 |
| 4 | L-tartaric acid | 1:1.1:1.1:3 | 79.5 | 97.6 |
| 5 | malic acid | 1:1.1:1.1:3 | 76.8 | 98.0 |
| 6 | succinic acid | 1:1.1:1.1:3 | 82.1 | 97.4 |
| 7 | oxalic acid | 1:1.1:1.1:3 | 79.8 | 98.1 |

The characterization data of the obtained compound are the same as those in Example 2.

### Example 8: Large-scale preparation

### a) Amide condensation

Compound of formula III (626 g, 1 mol), DMSO (3000 g) and PyBOP (572 g, 1.1 mol) were charged into a reaction tank, and stirred at 15^{~}25°C for 2 hours. TLC was used to monitor the time point when compound III disappeared. After the addition of organic acid salt IV (compound 2 dioxalate) (510 g, 1.1 mol), triethylamine (404.7 g, 4 mol) was added, and the mixture was stirred at 25°C for 2 hours. TLC was used to monitor the time point when the active ester intermediate state disappeared. Ethyl acetate (5008 g) and purified water (6260 g) were added and stirred for 30 minutes. The aqueous layer was removed out, and the organic layer was concentrated to dryness and directly used in the next step, yield being recorded as 100%.

### b) Hydrolysis

The freshly prepared compound of formula II (879 g, 1 mol) and a solution of acetone and ethanol (3516 g and 1758 g, respectively) were charged into a reaction flask, and then stirred homogeneously. A sodium hydroxide solution of 2 mol/L (158 g, 2020 mL) was added, and the reaction was carried out at 15^{~}25°C. TLC was used to monitor the time point when compound II disappeared. Then, a hydrochloric acid solution of 1 mol/L (396 g, 4040 mL) was begun to be added, the mixture was stirred at 10^{~}30°C to crystalize for 16^{~}18 hours, and 716.7 g white solid compound (formula I) was obtained after suction filtration, yield: 83.8%, purity: 98.3%. The characterization data of the obtained compound are the same as those in Example 2.

## Claims

1. A method for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base, **characterized in that**, the method comprises the following steps:
a) reacting compound of formula III with a condensation agent to give an active ester X; 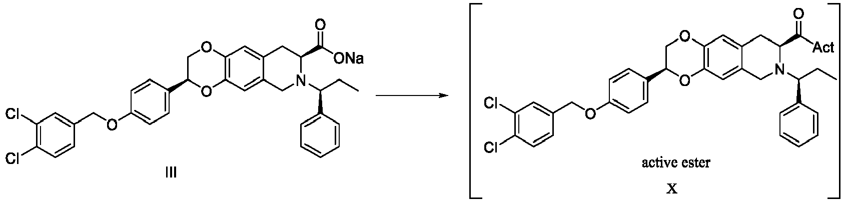
b) active ester X further reacting with organic acid salt IV to give compound of formula II, wherein x is 0.5^{~}3;

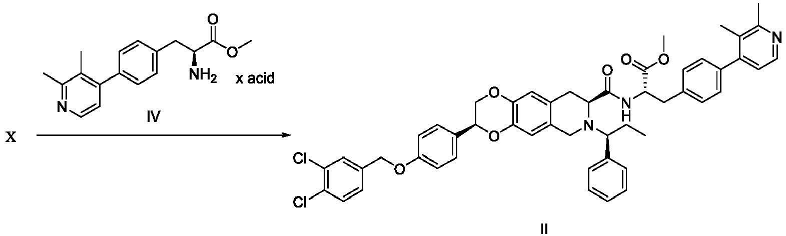

2. The method according to claim 1, **characterized in that**, the method further comprises the following step:
c) subjecting compound of formula II to hydrolysis and neutralization with an acid solution, to give compound of formula I;

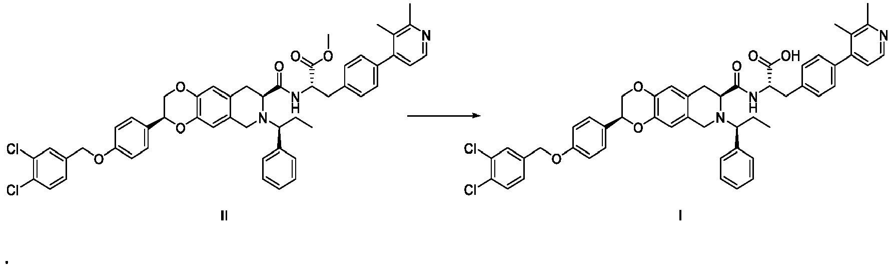

3. The method according to claim 1, **characterized in that**, the organic acid salt IV is selected from oxalate, citrate, L-tartrate, malate, succinate, maleate, fumarate, glycolate, hippurate, right Tosylate, benzoate or adipate.

4. The method according to claim 1 or 2, **characterized in that**, in step a), compound III is first reacted with a condensation agent to give an active ester, wherein the condensation agent is one, two or more selected from the group consisting of HATU, HBTU, HCTU, PyBOP, and TBTU, and preferably PyBOP; and/or, the active ester X is selected from: 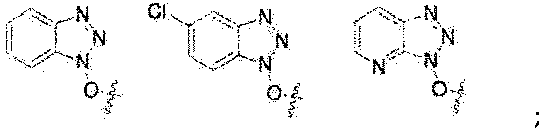 and/or compound III and the condensation agent are fed at a molar ratio in the range of 1:1.1^{~}1:2; and/or the reaction solvent in step a) is one, two or more selected from the group consisting of N,N-dimethyl formamide, N,N-dimethyl acetamide, dimethyl sulfoxide, dichloromethane, trichloromethane, tetrahydrofuran,and 2-methyltetrahydrofuran, and preferably dimethyl sulfoxide.

5. The method according to claim 1 or 2, **characterized in that**, in step a) after the addition of organic acid salt IV, alkaline reagent was addded, wherein the alkaline reagent is one, two or more organic bases selected from the group consisting of triethylamine, N-methylmorpholine, diisopropylethylamine, and 4-methylaminopyridine, and preferably triethylamine; and/or compound III, organic acid salt IV, and organic base are fed in a molar ratio in the range of 1:1.05:3^{~}1:1.1:5; and/or the organic acid salt IV is a salt formed from methyl (S)-2-amino-3-(4-(2,3-dimethylpyridin-4-yl)phenyl)propionate and organic acid, wherein the organic acid is one selected from the group consisting of oxalic acid, citric acid, L-tartaric acid, malic acid, succinic acid, maleic acid, fumaric acid, glycollic acid, and hippuric acid; and/or in step a), the reaction is carried out at a temperature in the range of 10^{~}50°C, and preferably in the range of 15^{~}35°C.

6. The method according to claim 1 or 2, **characterized in that**, the workup mode of step a) is as follows: after the completion of reaction, adding ethyl acetate and water are added in a weight amount 8^{~}10 times to that of compound III, or water alone is added in a weight amount 8^{~}10 times to that of compound III; the mixture is mixed homogeneously, extracted and washed; the aqueous layer is removed out, and the organic layer is concentrated to dryness and directly used in the next step, yield being recorded as 100%.

7. The method according to claim 2, **characterized in that**, in step b), the hydrolysis reaction is carried out by the addition of an aqueous solution of alkaline agent, wherein the alkaline reagent is one selected from lithium hydroxide, sodium hydroxide, and potassium hydroxide, and preferably potassium hydroxide, and the concentration of the aqueous solution is 1^{~}4 mol/L; and/or compound II and the alkaline reagent are fed at a molar ratio in the range of 1:2^{~}1:4; and/or the reaction solvent in step b) is one, two or more selected from the group consisting of tetrahydrofuran, 2-methyltetrahydrofuran, acetonitrile, acetone, methanol, and ethanol, and preferably acetone or a mixed solvent of acetone and ethanol; and/or in step b), the weight amount of the solvent is 4^{~}8 times to that of compound II, the weight ratio of acetone to ethanol in the mixed solvent is in the range of 4:1^{~}1:1, and preferably 2:1.

8. The method according to claim 2, **characterized in that**, in step b), the reaction mixture is neutralized with an acidic aqueous solution, wherein the acid is one selected from hydrochloric acid, hydrobromic acid, sulfuric acid, and phosphoric acid, and the concentration of the acidic aqueous solution is 1^{~}4mol/L.

9. The method according to claim 2, **characterized in that**, the workup mode of step b) is as follows: after the neutralization with an acid, the mixture is stirred at 10^{~}30°C to crystalize for 16^{~}20 hours.

10. The (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base obtained according to the method of any of claims 1-8, **characterized in that**, the purity of TTP273 free base is above 98%.

11. Use of the (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid free base obtained according to the method of any of claims 1-9 for preparing compound (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid dihydrochloride.
